(19) Europäisches Patentamt
European Patent Office
Office européen des brevets

(11) Veröffentlichungsnummer: **0 394 849 A1**

(12) **EUROPÄISCHE PATENTANMELDUNG**

(21) Anmeldenummer: **90107480.7**

(51) Int. Cl.5: **C07D 263/12, C07D 263/14**

(22) Anmeldetag: **19.04.90**

(30) Priorität: **28.04.89 DE 3914133**

(43) Veröffentlichungstag der Anmeldung: **31.10.90 Patentblatt 90/44**

(84) Benannte Vertragsstaaten: **GR**

(71) Anmelder: **Henkel Kommanditgesellschaft auf Aktien**
**Postfach 1100 Henkelstrasse 67**
**D-4000 Düsseldorf 1(DE)**

(72) Erfinder: **Fristad, William E., Dr.**
**Heinrich-Späth-Strasse 24**
**D-4019 Monheim(DE)**
Erfinder: **Leinen, Hans Theo, Dr.**
**Gertrudisstrasse 2**
**D-4000 Düsseldorf(DE)**
Erfinder: **Krause, Horst Jürgen, Dr.**
**Am Nettchesfeld 22**
**D-4000 Düsseldorf(DE)**
Erfinder: **Hase, Brigitte, Dr.**
**Gerhart-Hauptmann-Strasse 34**
**D-4006 Erkrath 6(DE)**
Erfinder: **Neumann, Peter**
**Nosthoffenstrasse 61**
**D-4000 Düsseldorf(DE)**

(54) **Verfahren zur Herstellung von 2-Oxazolinen.**

(57) Ein Verfahren zur Herstellung von 2-Oxazolinen, die in 2-Stellung mit aryl- bzw. aralkylsubstituenten $C_4$-$C_6$-Alkylgruppen, hydroxy- bzw. alkoxysubstituierten $C_1$-$C_6$-Alkylgruppen oder Alkoxyalkoxymethyl-Gruppen substituiert sind, ergibt die Titelverbindungen in hohen Ausbeuten, wenn man Carbonsäuren der Formel $R^1$-COOH, in denen $R^1$ einer der vorstehend genannten Substituenten ist, bzw. Ester oder Glyceride derselben mit 2-Aminoethanol oder Ethanolamide dieser Carbonsäuren in Gegenwart von Titan- bzw. Zirkoniumverbindungen des Typs $M(OR^3)_4$ (M = Ti bzw. Zr) umsetzt.

EP 0 394 849 A1

## Verfahren zur Herstellung von 2-Oxazolinen.

Die Erfindung betrifft ein Verfahren zur Herstellung von 2-Oxazolinen der allgemeinen Formel I

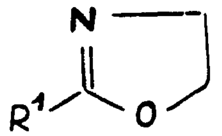

(I)

in der $R^1$
ein gegebenenfalls im aromatischen Kern substituierter Aryl-oder Aralkylrest,
eine Alkylgruppe mit 5 bis 6 Kohlenstoffatomen,
eine hydroxysubstituierte Alkylgruppe mit 1 bis 6 Kohlenstoffatomen,
eine alkoxysubstituierte Alkylgruppe mit 1 bis 6 Kohlenstoffatomen in der Alkyl- und 1 bis 18 Kohlenstoffatomen in der Alkoxygruppe, oder
einen Rest der allgemeinen Formel II
$R^2$-$(OC_2H_5)_p$-O-$CH_2$- (II)
in der
$R^2$ eine Alkyl- oder Alkenylgruppe mit 1 bis 18 Kohlenstoffatomen, insbesondere eine Methylgruppe, und
p eine Zahl im Bereich von 1 bis 10, insbesondere 1 bis 2, ist,
bedeutet,
durch Kondensation von Carbonsäureethanolamiden bzw. deren Vorläufern in Gegenwart von Katalysatoren in flüssiger Phase, wobei unter Vorläufern als erste Komponenten Carbon-säuren und Ester von Carbonsäuren mit niederen Alkanolen oder Glycerin und als zweite Komponente 2-Amino-ethanol zu verstehen sind.

2-Oxazoline mit einem Substituenten in 2-Stellung sind wertvolle Produkte, die unter anderem als Lösemittel oder Weichmacher und insbesondere als Polymerisationskomponenten eingesetzt werden.

Zur Herstellung dieser Verbindungsklasse sind zahlreiche Verfahren beschrieben worden:

Die einfachste Herstellung ist die Cyclodehydratisierung von N-2-Hydroxyethyl-carbonsäureamiden (Chem. Rev. 44, 447 ff (1949), Chem. Rev. 71, 485 ff (1971)). Die Cyclisierung der unsubstituierten N-2-Hydroxyethyl-carbonsäureamide erfordert jedoch sehr drastische Bedingungen oder die Anwesenheit spezieller Katalysatoren. Während zur Herstellung leicht flüchtiger, kurzkettiger 2-Alkyl-2-oxazoline Reaktionen in der Gasphase in Gegenwart von dehydratisierend wirkenden Metalloxiden wie $Al_2O_3$, $SiO_2/Al_2O_3$, $Al_2O_3/TiO_2$, $TiO_2$ oder MgO sich als geeignet erwiesen haben, stellt man die schwerer flüchtigen, längerkettigen 2-Alkyl-2-oxazoline besser in flüssiger Phase her. Als Katalysatoren für die Flüssigphasenreaktion sind Verbindungen des Mangans, Kobalts, Molybdäns, Wolframs, Eisens, Cadmiums, Zinks und Zinns sowie der seltenen Erdmetalle beschrieben worden (vgl. US-PS 3 562 263, BE-PS 666 829, C.A. 87, 135353, C.A. 87, 135352, US-PS 3 681 329, US-PS 3 681 333, EP-OS 00 33 752, US-PS 4 543 414, US-PS 4 354 029, US-PS 4 443 611, EP-OS 0 105 944 und EP-OS 0 164 219). Die in den vorgenannten Veröffentlichungen beschriebenen Katalysatoren führen bei der Darstellung längerkettiger 2-Fettalkyl-2-oxazoline jedoch nicht zu guten Ausbeuten.

Es wurde nun gefunden, daß sich spezielle Titan- und Zirkoniumverbindungen ausgezeichnet als Katalysatoren in einem Verfahren der eingangs genannten Art eignen, wobei Ausbeuten bis ca. 90 % der Theorie, bezogen auf die Ausgangsverbindung, erzielt werden können.

Demgemäß ist die Erfindung auf ein Verfahren der eingangs genannten Art gerichtet, bei dem man

a) Carbonsäuren der allgemeinen Formel III $R^1$-COOH (III)

in der $R^1$ wie oben definiert ist, Ester dieser Carbonsäuren mit Monoalkanolen mit 1 bis 4 Kohlenstoffatomen oder Glyceride dieser Carbonsäuren mit 2-Aminoethanol oder

b) Ethanolamide dieser Carbonsäuren in Gegenwart von Titan- bzw. Zirkoniumverbindungen der allgemeinen Formel IV $M(OR^3)_4$ (IV)

in der M vierwertiges Titan oder Zirkonium und $R^3$ eine Alkyl gruppe mit mindestens 2, insbesondere 2 bis 4 Kohlenstoffatomen, eine Acylgruppe mit mindestens 2, insbesondere 2 bis 10 Kohlenstoffatomen oder eine 2-Aminoethylenoxygruppe oder einen Rest eines beta-Diketons der allgemeinen Formel V bedeuten,
$R^4$- C =CH-CO-$R^5$ (V)
in der $R^4$ und $R^5$ gleiche oder verschiedene Reste aus der von Alkylgruppen mit 1 bis 4 Kohlenstoffatomen und gegebenenfalls in p-Stellung substituiertem Phenyl gebildeten Gruppe sind, wobei jeweils zwei der Gruppen $R^3$ zusammen aus dem zweibindigen Rest eines zweiwertigen Alkohols mit 2 bis 4 Kohlenstoffatomen bestehen können,
in Gegenwart von Titanylacetylacetonat oder
oder in Gegenwart von Kondensationsprodukten von Titan(IV) bzw. Zirkonium(IV)-tetraalkoxylaten der allgemeinen Formel IV, in der M und $R^3$ wie oben definiert sind, mit mehrfunktionellen Alkanolen, insbesondere mit 3 bis 12 Kohlenstoffatomen und 2 bis 6 Hydroxylgruppen,
kondensiert und die so erhaltenen 2-Oxazoline unter Entfernung des gebildeten Wassers und Alkohols bzw. Glycerins isoliert.

Als Ausgangsmaterial werden in dem Verfahren der Erfindung insbesondere die folgenden Carbonsäuren sowie deren Ester mit niederen Alkanolen oder Glycerin und deren Ethanolamide eingesetzt:
Benzoesäure, die 1 bis 3 Substituenten aufweisen kann; typische Substituenten sind $C_1$-$C_4$-Alkylgruppen, insbesondere Methyl, $C_1$-$C_4$-Alkoxygruppen, insbesondere Methoxy, sowie Halogenatome wie Chlor und Brom;
Phenylessigsäure oder Phenylpropionsäure, die im aromatischen Kern mit 1 bis 3 der vorgenannten Substituenten substituiert sein kann;
Capron- oder Önanthsäure;
Hydroxycarbonsäuren mit 2 bis 7 Kohlenstoffatomen, insbesondere Hydroxyessigsäure (Glykolsäure), Hydroxypropionsäure, Hydroxybuttersäure, Hydroxyvaleriansäure, Hydroxycapronsäure, insbesondere die omega-hydroxysubstituierten Isomeren derselben und gegebenenfalls deren Lactone;
alkoxysubstituierte Carbonsäuren, die formal Alkylierungsprodukte der vorgenannten Hydroxycarbonsäuren sind und 1 bis 18 Kohlenstoffatome in der Alkoxygruppe aufweisen; bevorzugt sind methoxysubstituierte Carbonsäuren;
Ethercarbonsäuren der Formel
$R^2$-$(OC_2H_5)_p$-O-$CH_2$-COOH
in der $R^2$ und p wie oben definiert sind; typische Beispiele hierfür sind Ethercarbonsäuren, die durch katalytische Oxidation von Anlagerungsprodukten von Ethylenoxid an primäre, gesättigte oder gegebenenfalls auch ungesättigte Alkohole mit 1 bis 18 Kohlenstoffatomen wie Methanol, Ethanol, Propanol, Butanol, Pentanol und Hexanol sowie gesättigte oder ungesättigte Fettalkohole einschließlich technischer Gemische derselben wie Capron-, Önanth-, Capryl-, Caprin-, Undecenyl-, Lauryl-, Myristyl-, Cetyl-, Stearyl-, Oleyl-, Elaidyl-, Linoleyl- und Linoleylalkohol, erhalten werden und z.B. in DE-A 26 36 123, EP-B 0 039 111, EP-B 0 018 681 und DE-A 31 35 946 beschrieben sind.

Besonders bevorzugte Ethercarbonsäuren sind Methoxy-ethoxy-und Methoxy-ethoxyethoxyessigsäure.

Die in dem Verfahren der Erfindung einsetzbaren Carbonsäureethanolamide können nach üblichen Verfahren erhalten werden, z.B. durch Umsetzung der Säurechloride oder der $C_1$-$C_4$-Alkylester der Carbonsäuren mit Ethanolamin.

Die als Katalysatoren im Verfahren der Erfindung einsetzbaren Titan- bzw. Zirkoniumverbindungen sind bekannt und größtenteils im Handel erhältlich. Kondensationsprodukte von Titan(IV) bzw. Zirkonium(IV)-tetraalkoxylaten mit mehrfunktionellen Alkanolen mit 3 bis 12 Kohlenstoffatomen und 2 bis 6 Hydroxylgruppen wie Glycerin, Trimethylolpropan und Pentaerythrit sind Veresterungs- und/oder Umesterungskatalysatoren, die z.B. in der US-C 4 705 764, auf deren Inhalt hier bezug genommen wird, beschrieben sind. Ein geeignetes Polyalkanol ist weiterhin Polyvinylalkohol. Die ebenfalls als Katalysatoren geeigneten Titan- bzw. Zirkoniumtetra-(2-aminoethoxylate) sind aus Titan- bzw. Zirkoniumtetraalkoxylaten und 2-Aminoethanol herstellbar.

Gemäß einer bevorzugten Ausführungsform der Erfindung verwendet man als Katalysatoren Ester der Titansäure ($H_4TiO_4$) oder der Zirkonsäure ($H_4ZrO_4$) bzw. gemischte Anhydride der Titan-oder Zirkonsäure mit organischen Säuren der allgemeinen Formel IV, in der M = Ti oder Zr und $R^3$ eine Alkylgruppe mit mindestens 2 oder mehr als 2, insbesondere 2 bis 4 Kohlenstoffatomen bzw. eine von einer Monocarbonsäure abgeleitete Acyl gruppe mit 2 oder mehr als 2, insbesondere 2 bis 10 Kohlenstoffatomen bedeuten.

Gemäß einer weiteren vorteilhaften Ausführungsform der Erfindung verwendet man Katalysatoren aus der von Titan- bzw. Zirkonium-tetraethylat, -tetrapropylat, -tetraisopropylat, -tetrabutylat und -tetraacetat gebildeten Gruppe.

Gemäß einer weiteren vorteilhaften Ausführungsform der Erfindung verwendet man als Katalysatoren Titan- bzw. Zirkoniumacetylacetonate der allgemeinen Formel (VI)
$(R^6O)_mM(ACA)_n$ (VI)
in der $R^6$ eine Alkylgruppe mit 1 bis 4 Kohlenstoffatomen, ACA einen Acetylacetonatrest und m die Zahl 0 und n die Zahl 4 oder m die Zahl 2 und n die Zahl 2 bedeuten.

Gemäß einer weiteren vorteilhaften Ausführungsform der Erfindung verwendet man als Katalysatoren Polykondensationsprodukte von Estern der Titansäure mit Monoalkanolen mit 2 bis 10 Kohlenstoffatomen mit Pentaerythrit.

Gemäß einer weiteren vorteilhaften Ausführungsform der Erfindung verwendet man die erfindungsgemäß einzusetzenden Katalysatoren in einer Menge von 0,1 bis 10, vorzugsweise 0,5 bis 5, insbesondere 0,5 bis 3 Mol-%, bezogen auf Carbonsäureethanolamid, bzw. die Vorläufer desselben.

Gemäß einer weiteren vorteilhaften Ausführungsform der Erfindung führt man die Kondensationsreaktion bei 150 bis 270 °C durch, wobei man die Reaktion vorzugsweise im Vakuum sowie unter Schutzgas durchführt. Man kann das gebildete Reaktionswasser zusammen mit den 2-Oxazolinen abdestillieren und von diesen während der Destillation trennen, wobei man mitgeschlepptes Restwasser, mit üblichen Trocknungsmitteln wie wasserfreiem Natriumsulfat oder Molekularsieb (4A) abtrennen kann. Es ist jedoch auch möglich, das Reaktionswasser durch azeotrope Destillation mittels hochsiedender Schleppmittel wie z.B. Tetralin oder Cumol aus dem Reaktionsgemisch vor der eigentlichen Destillation der 2-Oxazoline zu entfernen.

Gemäß einer weiteren bevorzugten Ausführungsform der Erfindung kann man die 2-Oxazoline direkt aus Carbonsäuren bzw. Carbonsäureestern der allgemeinen Formel VII

$R^1$-COOR$^7$ (VII)

in der $R^1$ wie oben definiert ist und $R^7$ Wasserstoff oder eine Alkylgruppe mit 1 bis 4 Kohlenstoffatomen bedeutet, oder aus Glyceriden der Carbonsäuren der Formel $R^1$-COOH, insbesondere Triglyceriden, herstellen, indem man diese in Gegenwart von Ethanolamin zu den Carbonsäureethanolamiden umsetzt und das Reaktionsgemisch anschließend in einer zweiten Reaktionsstufe unter weiterer Steigerung der Reaktionstemperatur reagieren läßt. Hierbei sind als Ausgangsprodukte die technisch leicht zugänglichen Carbonsäuremethylester und -glyceride bevorzugt. Diese setzt man in Gegenwart von Ethanolamin und den beanspruchten Katalysatoren in einer ersten Reaktionsstufe bei erhöhter Temperatur, vorzugsweise bei atmosphärischem Druck, unter Entfernung von Wasser und Alkohol bzw. Glycerin zu Carbonsäureethanolamiden um und läßt das Reaktionsgemisch in einer zweiten Reaktionsstufe unter weiterer Steigerung der Reaktionstemperatur, vorzugsweise im Vakuum, reagieren. Vorzugsweise setzt man das Ethanolamin dabei in einem 50 bis 400 mol-%-igen Überschuß, bezogen auf Ausgangsmaterial, ein, wobei man nicht umgesetztes bzw. überschüssiges Ethanolamin vor der zweiten Reaktionsstufe aus dem Reaktionsgemisch entfernt. In der ersten Reaktionsstufe verwendet man bevorzugt eine Reaktionstemperatur von 100 bis 170 °C und in der zweiten eine solche von 175 bis 250 °C. Die Katalysatoren werden bevorzugt in einer Menge von 0,1 bis 10, vorzugsweise 0,5 bis 5, insbesondere 0,5 bis 3 mol-%, bezogen auf eingesetztes Ausgangsmaterial, verwendet. Dabei werden wiederum bevorzugt Titantetraalkoholate, insbesondere ausgewählt von der aus Titantetraethylat, -tetrapropylat, -tetraisopropylat und -tetrabutylat gebildeten Gruppe, eingesetzt. Alternativ werden gemischte Anhydride der Titansäure mit Monocarbonsäuren, insbesondere solcher mit 1 bis 4 Kohlenstoffatomen, vorzugsweise Titantetraacetat, verwendet. Weiterhin hier bevorzugte Katalysatoren sind Titan- bzw. Zirkoniumacetylacetonate der allgemeinen Formel VI sowie die oben erwähnten Polykondensationsprodukte von Estern der Titansäure mit Monoalkanolen mit 2 bis 10 Kohlenstoffatomen mit Pentaerythrit sowie Titan (IV)-bzw. Zr(IV)-tetraaminoethanolate.

Bei dieser Verfahrensvariante führt man die Reaktion bevorzugt im Vakuum durch und destilliert das gebildete Reaktionswasser zusammen mit den 2-Oxazolinen unter weiterer Auftrennung während der Destillation, oder man entfernt vor der Destillation der 2-Oxazoline das Reaktionswasser durch azeotrope Destillation.

Besonders vorteilhaft ist es, hier die erste und zweite Reaktionsstufe als Ein-Topf-Verfahren in einem Reaktor durchzuführen.

Die Erfindung wird im folgenden anhand von bevorzugten Ausführungsbeispielen näher erläutert.

### Beispiel 1.

2-Phenyl-2-oxazolin.

In einer Rührapparatur mit Fraktionierkolonne, absteigendem Kühler mit Destillationsvorlagen, Vakuumeinrichtung mit Kühlfalle für das Reaktionswasser, Thermometer und Einleitungsrohr für Schutzgas wurde eine Mischung aus

1042 g (7,5 mol) Benzoesäuremethylester (98 %-ig),
921 g (15 mol) Ethanolamin (99,5 %-ig) und
25,5 g (0,075 mol, 1 mol-%) Titan-tetrabutylat
vorgelegt.

Das Reaktionsgemisch wurde unter Stickstoff erhitzt; das bei 128 bis 160 °C entstehende Methanol

wurde bei Normaldruck abdestilliert.

Anschließend wurde im Vakuum bei 87° C und 20 hPa das überschüssige Ethanolamin abdestilliert.

Das so erhaltene Benzoesäureethanolamid wurde im Vakuum auf 178 - 226° C erhitzt, wobei bei 122° C und 20 hPa das gebildete 2-Phenyl-2-oxazolin und Wasser gleichzeitig überdestillierten. Das Wasser wurde zum größten Teil in der Kühlfalle kondensiert. Um das überdestillierte Oxazolin von eventuell mitgerissenem Wasser zu befreien, wurde in die Destillationsvorlagen als Trockenmittel ein Molekularsieb (4 A) gegeben.

Man erhielt 836,7 g (75 % der Theorie, bezogen auf eingesetzten Benzoesäuremethylester) an 2-Phenyl-2-oxazolin, das noch geringe Mengen an Benzoesäureethanolamid enthielt.

Durch erneute Destillation am Dünnschichtverdampfer (Kp. = 120 bis 125° C bei 19 hPa) erhielt man 775,3 g (70 % der Theorie) an reinem 2-Phenyl-2-oxazolin mit den folgenden Kenndaten:
$n_D^{20}$ = 1,5672
IR: 1650 (C=N); 1260, 1065 (C-O-C=);975, 945, 900cm$^{-1}$ (Oxazolin).

Beispiel 2.

2-Hydroxymethyl-2-oxazolin.

Analog zu der in Beispiel 1 beschriebenen Arbeitsweise wurde Glykolsäuremethylester mit Ethanolamin umgesetzt. Die Ansatzmengen waren:
135,1 g (1,5 mol) Glykolsäuremethylester
184,2 g (3,0 mol) Ethanolamin (99,5 %-ig)
5,1 g (0,015 mol, 1 mol-%) Titan-tetrabutylat.
Ausbeute = 31,6 g.
Sublimationspunkt = 98° C (21 hPa).

Nach Umkristallisation erhielt man 20,7 g an reinem 2-Hydroxymethyl-2-oxazolin.

| Analyse: | |
|---|---|
| $C_4H_7NO_2$ (101,107) | |
| $C_{ber}$ = 47,52<br>$H_{ber}$ = 6,98<br>$N_{ber}$ = 13,85 | $C_{gef}$ = 47,5<br>$H_{gef}$ = 7,00<br>$N_{gef}$ = 13,6 |

IR: 1680 (C=N); 1209, 1099 (C-O-C=); 981, 956, 922cm$^{-1}$ (Oxazolin);
$^1$H-NMR: delta (in ppm) = 4,76 (1H,s), 4,24 (2H,t,J = 7 Hz), 3,86 (2H,t,J = 7 Hz).

Beispiel 3.

2-(5-Hydroxypentyl)-2-oxazolin.

Analog zu der Arbeitsweise des Beispiels 1 wurde Caprolacton mit Ethanolamin umgesetzt. Die Ansatzmengen waren:
230,6 g (2,0 mol) Caprolacton
245,5 g (4,0 mol) Ethanolamin (99,5 %-ig)
6,8 g (0,02 mol, 1 mol-%) Titan-tetrabutylat.
Ausbeute: 249,8 g (ca. 80 % Reinheit).
Kp = 140° C (0,03 hPa).

Nach erneuter Vakuumdestillation (Kp = 114° C bei 1,2 hPa) erhielt man 77,2 der Titelverbindung in 95 %-iger Reinheit.

Beispiel 4.

Methoxyethyl-2-oxazolin.

Analog zu der Arbeitsweise des Beispiels 1 wurde Methoxyessigsäure mit Ethanolamin umgesetzt. Die Ansatzmengen waren:
180,2 g (2,0 mol) Methoxyessigsäure (Reinheit über 95 %)
245,5 g (4,0 mol) Ethanolamin
6,8 g (0,02 mol, 1 mol-%) Titan-tetrabutylat:
Ausbeute = 188,6 g (82 % der Theorie).
Kp = 130 °C bis 163 °C (167 hPa).

Nach erneuter Vakuumdestillation erhielt man 124,4 g (54 % der Theorie) an reinem 2-Methoxymethyl-2-oxazolin; Kp. 69 °C/20 hPa.

| Analyse: | |
|---|---|
| $C_5H_9NO_2$ (115,134) | |
| $C_{ber}$ = 52,16 | $C_{gef}$ = 52,0 |
| $H_{ber}$ = 7,88 | $H_{gef}$ = 7,94 |
| $N_{ber}$ = 12,17 | $N_{gef}$ = 12,3 |

IR: 1668 (C=N); 1191, 1122 (C-O-C=); 982, 948, 918 $cm^{-1}$ (Oxazolin);
$^1$H-NMR: delta (in ppm) = 4,31 (2H,t,J = 7 Hz), 4,12 (2H,s), 3,90 (2H,t,J = 7 Hz), 3,46 (3H,s).

Beispiel 5.

2-(2,5-Dioxahexyl)-2-oxazolin.

Analog zu der Arbeitsweise des Beispiels 1 wurde 3,6-Dioxaheptansäure (Methoxyethoxyessigsäure) mit Ethanolamin umgesetzt.

Die Ansatzmengen waren:
235 g (1,50 mol) 3,6-Dioxaheptansäure (Reinheit ca. 80 - 85 %)
215 g (3 mol) Ethanolamin (99,5 %-ig)
6,0 g (0,0175 mol, 1 mol-%) Titantetrabutylat.
Ausbeute = 209,1 g (88 % der Theorie).
Kp = 140 bis 180 °C (0,5 hPa).

Nach erneuter Vakuumdestillation (Kp = 57 bis 66 °C bei 0,01 hPa) erhielt man 129,1 g (61 % der Theorie) der Titelverbindung.

| Analyse: | |
|---|---|
| $C_7H_{13}NO_3$ (159,187) | |
| $C_{ber}$ = 52,82 | $C_{gef}$ = 52,8 |
| $H_{ber}$ = 8,23 | $H_{gef}$ = 8,51 |
| $N_{ber}$ = 8,80 | $N_{gef}$ = 8,77 |

$n_D^{20}$ = 1,4571
IR: 1669 (C=N); 1199, 1116 (C-O-C=); 982, 950, 907 $cm^{-1}$ (Oxazolin);
$^1$H-NMR: delta (in ppm) = 4,30 (2H,t,J = 7 Hz), 4,24 (2H,s), 3,89 (2H,t,J = 7 Hz), 3,7446 (2H,dd,J = 3,4 Hz), 3,58 (2H,dd,J = 3,4 Hz), 3,38 (3H,s).

Beispiel 6.

2-(2,5,8-Trioxanonyl)-2-oxazolin.

Analog zu der Arbeitsweise des Beispiels 1 wurde 3,6,9-Trioxadecansäure (Methoxyethoxyethoxyessigsäure) mit Ethanolamin umgesetzt. Die Ansatzmengen waren:
231,6 g (1,24 mol) 3,6,9-Trioxadecansäure (Reinheit ca. 95 %)
159,6 g (2,6 mol) Ethanolamin (99,5 %-ig)
44 g (0,013 mol, 1 mol-%) Titan-tetrabutylat.
Ausbeute = 187,0 g (75 % der Theorie).
Kp = 167 - 182 °C (0,4 - 2,6 hPa).

Nach erneuter Vakuumdestillation (Kp = 101 - 106 °C bei 0,15 hPa) erhielt man 149,1 g (59 %) der reinen Titelverbindung.

| Analyse: | |
|---|---|
| $C_9H_{17}NO_4$ (203,24) | |
| $C_{ber}$ = 53,19<br>$H_{ber}$ = 8,43<br>$N_{ber}$ = 6,89 | $C_{gef}$ = 53,8<br>$H_{gef}$ = 8,49<br>$N_{gef}$ = 6,92 |

$n_D^{20}$ = 1,4602
IR: 1669 (C=N); 1198, 1109 (C-O-C=); 982, 950, 907 $cm^{-1}$ (Oxazolin);
$^1$H-NMR: delta (in ppm)= 4,30 (2H,t,J = 7 Hz), 4,23 (2H,s), 3,88 (2H,t,J = 7 Hz), 3,75 (2H,m), 3,70 (2H,m), 3,65 (2H,m), 3,55 (2H,m), 3,38 (3H,s).

Beispiel 7.

Herstellung eines 2-substituierten-2-oxazolins aus m-Tolylsäure.

Analog der Herstellung zum Beispiel 1 wurde m-Tolylsäure mit Ethanolamin zum 2-(3-Methylphenyl)-2-oxazolin umgesetzt. Die Ansatzmengen waren:
347,3 g (2,5 mol) m-Tolylsäure, 98 %-ig
307,0 g (5,0 mol) Ethanolamin, 99,5 %-ig
25,5 g (0,075 mol, 3 mol-%) Titan-tetrabutylat
Ausbeute = 241 g (60 % d. Th.) 2-(3-Methylphenyl)-2-oxazolin.
KP = 128 - 142 °C (16 hPa).
IR: 1650 (C=N); 1268, 1067 (C-O-C=); 979, 951, 910 $cm^{-1}$ (Oxazolin).
$^1$H-NMR: delta (in ppm) 7,78 (1H,s); 7,72 (1H,m); 7,29 (2H,m); 4,42 (2H,t,J = 7 Hz); 4,04 (2H,t,J = 7 Hz); 2,37 (3H,s).

Beispiel 8.

Herstellung eines 2-substituierten-2-oxazolins aus m-Methoxybenzoesäure.

Analog der Herstellung zum Beispiel 1 wurde m-Methoxybenzoesäure mit Ethanolamin zum 2-(3-Methoxyphenyl)-2-oxazolin umgesetzt. Die Ansatzmengen waren:
232,9 g (1,5 mol) m-Methoxybenzoesäure, 98 %-ig
184,2 g (3,0 mol) Etanolamin, 99,5 %-ig
15,3 g (0,045 mol, 3 mol-%) Titan-tetrabutylat
Ausbeute = 116 g (44 % d. Th.) 2-(3-Methoxyphenyl)-2-oxazolin.
Kp = 122 - 157 °C (17-19 hPa)
IR: 1650 (C=N); 1270, 1043 (C-O-C=); 980, 951, 910 $cm^{-1}$ (Oxazolin).
$^1$H-NMR: delta (in ppm) 7,54 (1H,dt,J = 6; 1 Hz); 7,50 (1H,t,J = 1 Hz); 7,32 (1H,t,J = 6 Hz); 7,03 (1H,dt,J

= 6; 1 Hz); 4,43 (2H,t,J = 7 Hz); 4,06 (2H,t,J = 7 Hz); 3,86 (3H,s).

Beispiel 9.

Herstellung von 2-Phenyl-2-oxazolin aus Benzoesäuremethylester in Gegenwart von Titan(IV)-diisopropoxy-bis-acetylacetonat.

Analog der Herstellung zum Beispiel 1 wurde Benzoesäuremethylester mit Ethanolamin zum 2-Phenyl-2-oxazolin umgesetzt. Die Ansatzmengen waren:
272,3 g (2,0 mol) Benzoesäuremethylester, 98 %
244,3 g (4,0 mol) Ethanolamin, 99,5 %-ig
7,3 g (0,02 mol, 1 mol-%) Titan(IV)-diisopropoxy-bis-acetylacetonat.
Ausbeute = 236 g (82 % d. Th.) 2-Phenyl-2-oxazolin.
Kp = 107 - 120 °C (20 hPa)

Beispiel 10.

Herstellung von 2-Phenyl-2-oxazolin aus Benzoesäureethanolamid in Gegenwart von Titan-tetrabutylat.

Analog der Herstellung zum Beispiel 1 (zweite Stufe) wurde Benzoesäureethanolamid zum 2-Phenyl-2-oxazolin umgesetzt. Die Ansatzmengen waren:
330,4 g (1,9 mol) Benzoesäureethanolamid, 95 %
7,3 g (0,02 mol, 3 mol-%) Titan-tetrabutylat
Ausbeute = 216 g (77 % d. Th.) 2-Phenyl-2-oxazolin.
Kp = 112 - 114 °C (19 hPa).

**Ansprüche**

1. Verfahren zur Herstellung von 2-Oxazolinen der allgemeinen Formel I

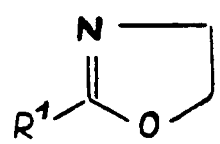

(I)

in der $R^1$
ein gegebenenfalls im aromatischen Kern substituierter Aryl- oder Aralkylrest,
eine Alkylgruppe mit 4 bis 6 Kohlenstoffatomen,
eine hydroxysubstituierte Alkylgruppe mit 1 bis 6 Kohlenstoffatomen,
eine alkoxysubstituierte Alkylgruppe mit I bis 6 Kohlenstoffatomen in der Alkyl- und 1 bis 18 Kohlenstoffatomen in der Alkoxygruppe, oder
einen Rest der allgemeinen Formel II
$R^2$-$(OC_2H_5)_p$-O-$CH_2$- (II)
in der
$R^2$ eine Alkyl- oder Alkenylgruppe mit 1 bis 18 Kohlenstoffatomen, insbesondere eine Methylgruppe, und
p eine Zahl im Bereich von 1 bis 10, insbesondere 1 bis 2, ist,
bedeutet,
durch Kondensation von Carbonsäureethanolamiden bzw. deren Vorläufern in Gegenwart von Katalysatoren in flüssiger Phase,
dadurch gekennzeichnet, daß man
a) Carbonsäuren der allgemeinen Formel III
$R^1$-COOH (III)

in der $R^1$ wie oben definiert ist, Ester dieser Carbonsäuren mit Monoalkanolen mit 1 bis 4 Kohlenstoffatomen oder Glyceride dieser Carbonsäuren mit 2-Aminoethanol oder

b) Ethanolamide dieser Carbonsäuren

in Gegenwart von Titan- bzw. Zirkoniumverbindungen der allgemeinen Formel IV

$M(OR^3)_4$ (IV)

in der M vierwertiges Titan oder Zirkonium und $R^3$ eine Alkylgruppe mit mindestens 2, insbesondere 2 bis 4 Kohlenstoffatomen, eine Acylgruppe mit mindestens 2, insbesondere 2 bis 10 Kohlenstoffatomen oder eine 2-Aminoethy lenoxygruppe oder einen Rest eines beta-Diketons der allgemeinen Formel V bedeuten,

$R^4$- C =CH-CO-$R^5$ (V)

in der $R^4$ und $R^5$ gleiche oder verschiedene Reste aus der von Alkylgruppen mit 1 bis 4 Kohlenstoffatomen und gegebenenfalls in p-Stellung substituiertem Phenyl gebildeten Gruppe sind, wobei jeweils zwei der Gruppen $R^3$ zusammen aus dem zweibindigen Rest eines zweiwertigen Alkohols mit 2 bis 4 Kohlenstoffatomen bestehen können,

in Gegenwart von Titanylacetylacetonat oder

in Gegenwart von Kondensationsprodukten von Titan(IV) bzw. Zirkonium(IV)-tetraalkoxylaten der allgemeinen Formel IV, in der M und $R^3$ wie oben definiert sind, mit mehrfunktionellen Alkanolen, insbesondere mit 3 bis 12 Kohlenstoffatomen und 2 bis 6 Hydroxylgruppen,

kondensiert und die so erhaltenen 2-Oxazoline unter Entfernung des gebildeten Wassers und Alkohols bzw. Glycerins isoliert.

2. Verfahren nach Anspruch 1, dadurch gekennzeichnet, daß man als Katalysatoren Ester der Titansäure ($H_4TiO_4$) oder Zirkonsäure ($H_4ZrO_4$) bzw. gemischte Anhydride der Titansäure oder Zirkonsäure mit organischen Säuren der allgemeinen Formel IV, in der M = Ti oder Zr und $R^3$ eine Alkylgruppe mit 2 oder mehr als 2, insbesondere 2 bis 4 Kohlenstoffatomen bzw. eine von einer Monocarbonsäure abgeleitete Acylgruppe mit 2 oder mehr als 2, insbesondere 2 bis 10 Kohlenstoffatomen bedeuten, verwendet.

3. Verfahren nach Anspruch 1 oder 2, dadurch gekennzeichnet, daß man Katalysatoren aus der von Titan- bzw. Zirkonium-tetraethylat, -tetrapropylat, -tetraisopropylat, -tetrabutylat und -tetraacetat gebildeten Gruppe verwendet.

4. Verfahren nach Anspruch 1 oder 2, dadurch gekennzeichnet, daß man Tetra(aminoethyl)-titanat oder -zirkonat als Katalysator verwendet.

5. Verfahren nach Anspruch 1 oder 2 , dadurch gekennzeichnet, daß man als Katalysatoren Titan- bzw. Zirkoniumacetylacetonate der allgemeinen Formel VI

$(R^6O)_mM(ACA)_n$ (VI)

in der $R^6$ eine Alkylgruppe mit 1 bis 4 Kohlenstoffatomen,

ACA einen Acetylacetonatrest und

m die Zahl 0 und n die Zahl 4 oder m die Zahl 2 und n die Zahl 2

bedeuten, verwendet.

6. Verfahren nach Anspruch 1 oder 2, dadurch gekennzeichnet, daß man als Katalysatoren Polykondensationsprodukte von Estern der Titansäure mit Monoalkanolen mit 2 bis 10 Kohlenstoffatomen mit Pentaerythrit verwendet.

7. Verfahren nach mindestens einem der Ansprüche 1 bis 6, dadurch gekennzeichnet, daß man die Katalysatoren in Mengen von 0,1 bis 10, vorzugsweise 0,5 bis 5, insbesondere 0,5 bis 3 Mol-%, bezogen auf Carbonsäuremonoethanolamid bzw. Vorläufer desselben, verwendet.

8. Verfahren nach mindestens einem der Ansprüche 1 bis 7, dadurch gekennzeichnet, daß man die Kondensation bei 150 bis 270 °C durchführt.

9. Verfahren nach mindestens einem der Ansprüche 1 bis 8, dadurch gekennzeichnet, daß man die Reaktion im Vakuum durchführt.

10. Verfahren nach mindestens einem der Ansprüche 1 bis 9, dadurch gekennzeichnet, daß man das gebildete Reaktionswasser bzw. -alkanol zusammen mit den 2-Oxazolinen abdestilliert und anschließend oder gleichzeitig von diesen trennt oder vor der Destillation der 2-Oxazoline durch azeotrope Destillation entfernt.

11. Verfahren nach mindestens einem der Ansprüche 1 bis 10, dadurch gekennzeichnet, daß man in einer ersten Reaktionsstufe Carbonsäuren bzw. Carbonsäureester der allgemeinen Formel VII

$R^1$-$COOR^7$ (VII)

in der $R^1$ wie oben definiert ist und $R^7$ Wasserstoff oder eine Alkylgruppe mit 1 bis 4 Kohlenstoffatomen bedeutet, oder Carbonsäureglyceride in Gegenwart von Ethanolamin bei erhöhter Temperatur und bei atmosphärischem Druck zu den Fettsäureethanolamiden umsetzt, gebildetes Wasser und Alkohol bzw. Glycerin entfernt und das Reaktionsgemisch in einer zweiten Reaktionsstufe unter weiterer Steigerung der Reaktionstemperatur im Vakuum reagieren läßt.

12. Verfahren nach Anspruch 11, dadurch gekennzeichnet, daß man das Ethanolamin in einem 50 bis 400 Mol-%-igem Überschuß, bezogen auf eingesetzte Carbonsäure, Carbonsäureester oder Carbonsäureglyceride, verwendet.

13. Verfahren nach Anspruch 11 oder 12, dadurch gekennzeichnet, daß man vor der zweiten Reaktionsstufe nicht umgesetztes bzw. überschüssiges Ethanolamin aus dem Reaktionsgemisch entfernt.

14. Verfahren nach mindestens einem der Ansprüche 11 bis 13, dadurch gekennzeichnet, daß man in der ersten Reaktionsstufe eine Reaktionstemperatur von 100 bis 170 °C verwendet.

15. Verfahren nach mindestens einem der Ansprüche 11 bis 14, dadurch gekennzeichnet, daß man in der zweiten Reaktionsstufe eine Reaktionstemperatur von 175 bis 270 °C verwendet.

16. Verfahren nach mindestens einem der Ansprüche 11 bis 15, dadurch gekennzeichnet, daß man als Titansäureester Titantetraalkoholate verwendet.

17. Verfahren nach mindestens einem der Ansprüche 11 bis 16, dadurch gekennzeichnet, daß man Titantetraalkoholate aus der von Titantetraethylat, -tetrapropylat, tetraisopropylat und -tetrabutylat gebildeten Gruppe verwendet.

18. Verfahren nach mindestens einem der Ansprüche 11 bis 15, dadurch gekennzeichnet, daß man Tetra(aminoethyl)-titanat bzw. -zirkonat als Katalysator verwendet.

19. Verfahren nach mindestens einem der Ansprüche 11 bis 15, dadurch gekennzeichnet, daß man gemischte Anhydride der Titansäure mit Monocarbonsäuren verwendet.

20. Verfahren nach mindestens einem der Ansprüche 11 bis 15, dadurch gekennzeichnet, daß man Titantetraacetat verwendet.

21. Verfahren nach mindestens einem der Ansprüche 11 bis 15, dadurch gekennzeichnet, daß man als Katalysatoren Titan-bzw. Zirkoniumacetylacetonate der allgemeinen Formel VI

$(R^6O)_mM(ACA)_n$ (VI)

verwendet, in der $R^6$, ACA, m und n wie oben definiert sind.

22. Verfahren nach mindestens einem der Ansprüche 11 bis 15, dadurch gekennzeichnet, daß man als Katalysatoren Polykondensationsprodukte von Estern der Titansäure mit Monoalkanolen mit 2 bis 10 Kohlenstoffatomen mit Pentaerythrit verwendet.

23. Verfahren nach einem der Ansprüche 11 bis 22, dadurch gekennzeichnet, daß man die Katalysatoren in einer Menge von 0,1 bis 10, vorzugsweise 0,5 bis 5, insbesondere 0,5 bis 3 Mol-%, bezogen auf Carbonsäuren, Carbonsäureester bzw. Carbonsäureglyceride, zusetzt.

24. Verfahren nach mindestens einem der Ansprüche 11 bis 23, dadurch gekennzeichnet, daß man das in der zweiten Reaktionsstufe gebildete Reaktionswasser zusammen mit den 2-Oxazolinen abdestilliert und anschließend oder gleichzeitig entfernt oder vor der Destillation der 2-Oxazoline durch azeotrope Destillation entfernt.

25. Verfahren nach mindestens einem der Ansprüche 11 bis 24, dadurch gekennzeichnet, daß man die erste und zweite Reaktionsstufe als Ein-Topf-Verfahren durchführt.

Europäisches Patentamt

# EUROPÄISCHER RECHERCHENBERICHT

Nummer der Anmeldung

EP 90 10 7480

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | CHEMICAL ABSTRACTS, Band 87, Nr. 17, 24. Oktober 1977, Seite 729, Zusammenfassung Nr. 135352h, Columbus, Ohio, US; & JP-A-77 19 661 (MITSUI PETROCHEMICAL INDUSTRIES, LTD) 15-02-1977<br>* Zusammenfassung *<br>--- | 1 | C 07 D 263/12<br>C 07 D 263/14 |
| A | DE-B-1 094 749 (ROHM AND HAAS)<br>* Insgesamt *<br>--- | 1 | |
| D,A | US-A-3 681 329 (MORTON H. LITT)<br>* Patentansprüche *<br>--- | 1 | |
| P,X | EP-A-0 315 856 (HENKEL KGaA)<br>* Patentansprüche *<br>----- | 1-25 | |
| | | | RECHERCHIERTE SACHGEBIETE (Int. Cl.5) |
| | | | C 07 D 263/00 |

Der vorliegende Recherchenbericht wurde für alle Patentansprüche erstellt

| Recherchenort | Abschlußdatum der Recherche | Prüfer |
|---|---|---|
| DEN HAAG | 22-06-1990 | HENRY J.C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)